Europäisches Patentamt

European Patent Office

Office européen des brevets

(11) Publication number: **0 322 102**
**A2**

## EUROPEAN PATENT APPLICATION

(21) Application number: 88310725.2

(22) Date of filing: 14.11.88

(51) Int. Cl.⁴: **G01N 33/543** , //G01N33/569

(30) Priority: 30.11.87 US 126735

(43) Date of publication of application:
28.06.89 Bulletin 89/26

(84) Designated Contracting States:
AT BE CH DE ES FR GB GR IT LI LU NL SE

(71) Applicant: McCue, John P.
114 Union Park Street No. 3
Boston Massachusetts 02118(US)

(72) Inventor: McCue, John P.
114 Union Park Street No. 3
Boston Massachusetts 02118(US)

(74) Representative: Bizley, Richard Edward et al
BOULT, WADE & TENNANT 27 Furnival Street
London EC4A 1PQ(GB)

(54) Methods of improved antigen capture assays and compounds therefor.

(57) Method is provided for improving the amount of antibodies absorbed on surfaces and for improving the ability of surface-bound antibodies to capture antigens, by allowing the antibodies to formulate and absorb from solutions in the pH range 3.5 to 5.5. Method is also provided for improving the ability of (labeled) antibodies linked to markers to bind to antigens, by using antibodies that are formulated in the pH range 3.5 to 5.5. The invention further includes the improved antibody-coated surfaces that result from such method, as well as the improved labeled antibodies and the antibody-antigen-labeled antibody sandwich.

EP 0 322 102 A2

## METHODS OF IMPROVED ANTIGEN CAPTURE ASSAYS AND COMPOUNDS THEREFOR

This invention relates to antigen capture assays, particularly the improvement of such assays in detecting antigens.

Antigen-capture assays make use of the specific binding affinity of antibodies in order to detect antigens in clinical and research samples. As discussed herein, an "antigen" is any molecule that will induce an antibody response from cells or organisms and "antibodies" are globular proteins which specifically recognize and bind the antigens which induce their production by the cells or organism.

By immobilizing antibodies on a surface, the antibody-coated surface becomes capable of binding and retaining antigens specific to the antibodies. If antigens specific to these immobilized antibodies are present in a test solution, the antibody-coated surface will to some degree be able to capture antigens from the solution. Assays that utilize this principle are called antigen-capture assays.

The ability of antibodies to recognize and bind their antigens has been used for more than 30 years to test for the presence of specific antigens. Antigen capture assays are very important to medicine and the biotechnology industry, since such assays have the ability to detect and quantitate specific molecules of interest from very complex mixtures.

How well an antigen capture assay can detect the presence of a particular antigen is determined by how well the immobilized antibodies bind and retain small amounts of the antigen, and how specific the antibody binding is for the antigen of interest.

Important among such areas of application and by way of example, is the use of these assays for monitoring the course of viral infection. The importance of this application can be seen in the present situation involving organ transplant patients and Cytomegalovirus (CMV). Exposure to CMV is widespread among individuals in the United States as indicated by the percentage of people showing antibodies to the virus, but the percentage of these who actually carry the virus is unknown because the virus has a tendency to be hidden from the relatively insensitive tissue culture assays and antigen capture assays of the prior art. In the average individual the presence of CMV is not accompanied by known pathology and is therefore assumed to be without consequence, but in an individual who becomes immunosurpressed, such as an organ transplant patient, the presence of CMV becomes life threatening. To be able to reliably test for the presence of the virus is an important tool for the management of this problem in organ transplant medicine. Similarly with Human Immunodeficiency Virus (HIV), the etiological agent for AIDS, testing for the presence of this virus is done indirectly by testing for the presence of antibodies to HIV. However, such testing cannot tell if the virus is present. Thus, it is difficult to determine what percent of antibody positive people carry the virus, what percent is likely to go on to develop AIDS, or what the long term benefits of new therapies will be.

For any antigen capture assay to be useful for addressing these questions, the assay must be able to detect the presence of very small amounts of virus. Ideally the assay should be able to "find" a single replicating virus if it exists anywhere in the patient. However, because of sampling limits, this ideal cannot be attained. Nonetheless, assays which approach the ideal are extremely valuable for assessing the likelihood of active infection and measuring the relative change in patient status. This invention includes a new way of enhancing the ability of antigen capture assays to approach the ideal by improving the sensitivity of such assays.

Immobilization of antibodies on a surface is an important first step in the preparation of an antigen capture assay. The simplest, most convenient, and most commonly used method for immobilizing antibodies on surfaces is to allow them to spontaneously absorb on plastio surfaces. In the earliest studies on absorption of antibodies Oreskes and Singer (J. Immunology, 1961, vol. 86, pp 338-344) reported that purified proteins bind best to plastics at the protein isoelectric point. For antibodies of the IgG class this encompasses a pH range from about 6.5 to 9.5. This work has lead to the generally accepted theoretical model of how antibodies are absorbed, namely that they are absorbed by hydrophobic interactions, and that the largest number of antibody molecules that can be accommodated on a surface occurs at their isoelectric point where the least charge repulsion between molecules exists. In practice, these findings were confirmed by Catt and Tregear (Science, 1967, vol. 158, pp 1570-1572) when they reported that the optimal pH for absorbing antibodies on plastic surfaces was 9.0 to 10.0.

Thus, the tradition and practice that has evolved over the years is to allow absorption of antibodies to occur in the pH range 6.5 to 9.5. This practice has gone unchallenged not only because of the work of Catt & Tregear and Oresker & Singer, but also because of the belief that antibodies are unstable in solution at pH outside the physiological range (see:- P. Tijssen, Practice and Theory of Enzyme Immunoassys,

2

published by Elsevier, 1985). However, recently it was shown that purified antibodies are unstable in the physiological pH range (see: J.P. McCue, R. H. Hein & R. Tenold, Reviews of Infectious Diseases, vol. B, suppl. 4, pp s374-s38l, l986; and P. K. Sasagawa, J. P. McCue, R.H. Hein & R. Tenold, Transfusion, vol. 86, p 557, 1986).

In terms of optimizing antigen capture assays this created a dilemma: If purified antibodies are unstable at physiological pH but are best absorbed by surfaces at this pH, how does one optimize such assays? The dilemma became more troublesome with the report that optimal binding of antibody to antigen occurs in solution near the isoelectric point of the antibody (as published by Y. Endo, K. Miyai, N. Hata & Y. Iijima, Biotechnology & Applied Biochemistry, vol. 9, pp 74-81, 1987). Because of this, an investigation of the whole question of what constitutes optimal conditions for the formulation of antibodies for antigen capture assays was undertaken. Also there is a need and market for antigen capture assays that would improve upon the above prior art shortcomings, e.g. a need for improved sensitivity of assays for the detection of viruses.

There has now been discovered an improved method of antigen capture assays by improving the ability of antibodies to bind to surfaces with improved ability of the surface-bound antibodies to capture antigens and provide a more sensitive antigen test and thus improved testing for the presence of e.g. virus in humans.

Broadly, the present invention provides a method for improving antigen capture assays by formulating antibodies in the pH range of 3.5 to 5.5 and absorbing said antibodies on at least one surface in said pH range to form a more thoroughly antibody coated surface.

The above method seeks to bind more antibodies to surfaces and in at least some embodiments, to improve the ability of such surface-bound antibodies to capture antigens and provide a more sensitive test for small amounts of antigens.

The invention further provides method for improving the ability of labeled antibodies to bind to the so-captured antigens by using antibodies that are formulated in the pH range 3.5 to 5.5, thus improving the sensitivity of the test for the so-captured antigens.

In each case, the above antibodies are preferably isolated in solution in the pH range of 3.5 to 5.5 and thereafter formulated in that pH range. However, if desired, such antibodies can be isolated at a pH range above 5.5, e.g. in the pH range 5.5 to 9.5, as long as such pH is then promptly (within a few hours or sooner) lowered to formulate such antibodies in the pH range of 3.5 to 5.5, within the scope of the present invention.

For the purposes of the present invention, "antibodies", as used herein, includes monoclonal antibodies (of singular antigen specificity) and polyclonal antibodies (a mixture of antibodies of different antigen specificity). Such antibody formulations can be polyclonal, monoclonal or mixtures thereof, as desired, within the scope of the present invention.

The invention will become more apparent from the following-detailed specification and drawings in which;

Figure 1 is a schematic diagram showing antigen capture and detection according to a method embodying the present invention and

Figures 2, 3 and 4 show graphs illustrating the improved performance of antigen capture essays according to the present invention.

As noted above, the method of the present invention relates to improved antigen capture assays which in turn provide more sensitive tests for the presence of antigens e.g. virus in humans.

To demonstate the present invention, antibodies are isolated e.g. from human plasma and formulated under two different pH conditions. In a) a pH range of 3.5 to 5.5, according to methods of the present invention, and b) a pH of about 7.4, in keeping with the prior art. The respective two antibody solutions are then applied to wells of plastic (e.g. polystyrene) surfaces, allowed to sit e.g. for 48 hours, the solutions are then removed and the wells washed, which results in residual antibodies being immobilized on the plastic well surfaces.

Thereafter the wells are contacted with an antigen solution, the antigens of which, bind preferentially to the antibodies absorbed on the plastic surfaces. The amount of antigen retained by these surfaces is proportional (in part) to the amount of surface covered by the antibodies. The greater the coverage of the surface with antibodies, the more sensitive is the test for capturing small amounts of antigens, including virus, e.g. in human blood, for examples, CMV virus and HIV virus.

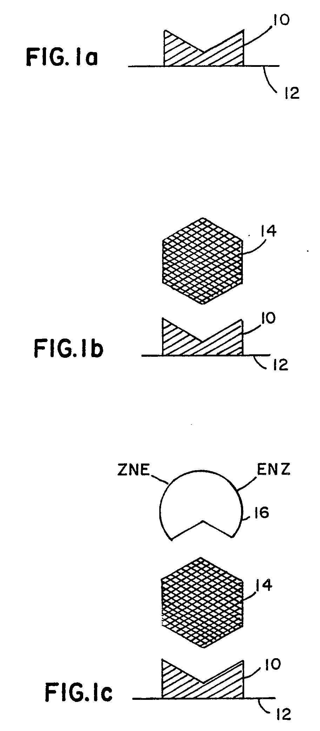

The excess antigen solution is then washed from the so-coated plastic surfaces and the captured antigens detected by contacting such coated surface with a second solution of "labeled" antibodies which bind in turn to the captured antigens. Labeling can be done with a number of antibodies to which are

3

EP 0 322 102 A2

attached radioactive isotopes, enzymes or fluorescent markers, e.g. as shown in Figure 1.

That is, the immobilized specific antibody 10 is coated on a plastic surface 12 and thereafter binds or captures an antigen 14, as shown in steps a) and b) respectively of Figure 1. Thereafter a labeled antibody 16 is contacted with the so-bound antibody 14, (e.g. in an antibody-antigen-labeled antibody sandwich), which enables visual and/or quantitative determination of the kind and number of antigens captured by the underlying antibodies coated earlier on the plastic surface.

The invention is believed to provide a method for a striking improvement in antigen capture assays which includes the novel methods outlined below:

a) Method for increasing the amount of antibodies absorbed on surfaces and also for improving the ability of surface-bound antibodies to capture antigens, by allowing the antibodies to formulate and absorb from solutions in the pry range of 3.5 to 5.5.

b) Method for improving the ability of antibodies linked to markers, i.e. labeled antibodies to bind antigens by using antibodies that are formulated in the pH range of 3.5 to 5.5 for more sensitive detection of such antigens.

The invention also includes the improved products resulting from such novel methods, including improved antibody-coated surfaces for antigen capture essays, as well as improved labeled antibodies for detecting the presence of the so-captured antigens.

The various pH ranges referred to herein relative to the prior art and the present invention include those tabulated below:

TABLE I

| pH Ranges | | |
|---|---|---|
| Process | Prior Art | The Invention |
| Antibody Isolation | pH Usually at 6.5 to 9.5 | pH 3.5 to 5.5 (and higher as discussed above) |
| Antibody Formulation | pH 6.5 to 9.5 | pH 3.5 to 5.5 |
| Antibody Absorption (Onto Surface) | pH 6.5 to 9.5 | pH 3.5 to 5.5 |
| Antibody-Antigen Reaction | Isolectric point | Isolectric point |
| Antibody-Label Coupling | Varies with chemistry of coupling reagent | Varies with chemistry of coupling reagent |
| Labeled Antibody-Antigen Reaction | pH near isolectric point | pH near isolectric point |

As indicated in the above Table, the present invention includes antibodies isolated and formulated at pH 3.5 to 5.5 and absorbed on a surface at such pH. However, the antibody isolation can take place at a higher pH as indicated and discussed above. Also included in the present invention are antibodies formulated at pH 3.5 to 5.5 which are then coupled (e.g. at higher pH, such as pH 5.5 to 9.5 or higher) to form labeled antibodies, which labeled antibodies are then stored at pH 3.5 to 5.5 as discussed above.

The pH of the isolectric point referred to in the above table is the pH range where the antibody-antigen charge repulsion is minimum, usually between pH 6.5 to 9.5 but in some cases may be above or below such range.

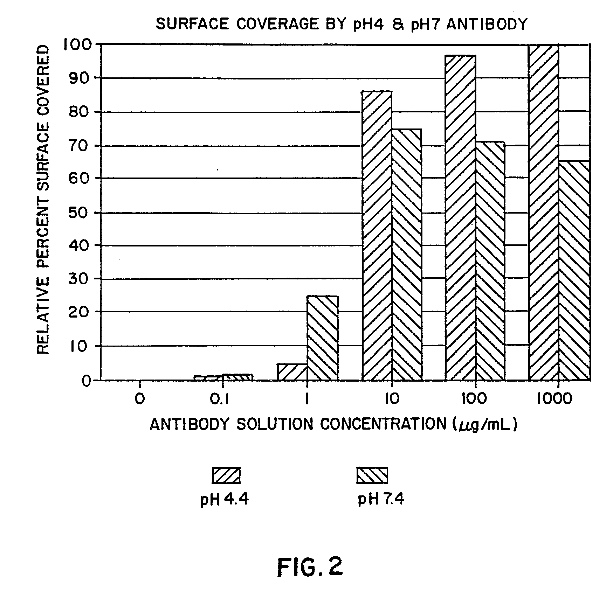

The improvements in the methods of the invention are illustrated, for example in Figures 2, 3 and 4 of the accompanying drawings as follows:

Figure 2 is a graph showing the relative amount of surface covered by antibodies absorbed from solutions at pH 4.4 and pH 7.4 and it shows that antibodies isolated and formulated near pH 7 were able to cover no more than 75% of the surface covered byantibodies isolated and formulated near pH 4;

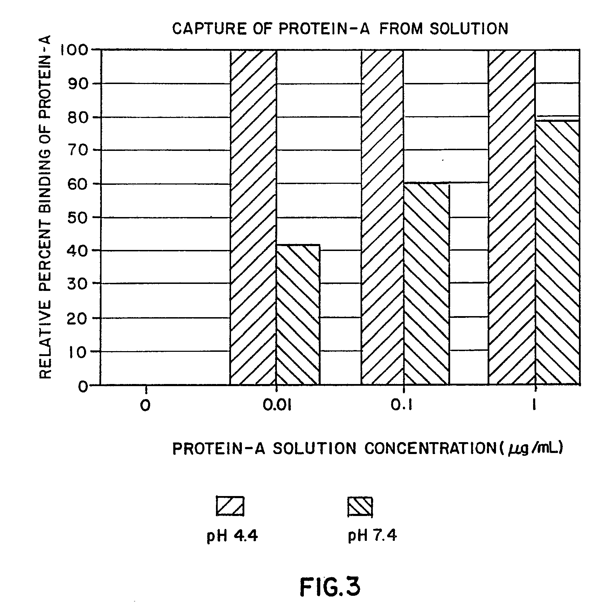

Figure 3 shows the improved ability of antibodies absorbed on surfaces at pH 4.4 to capture protein-A from solutions at various concentrations compared with the same antibodies absorbed on the surface at pH 7.4 and

4

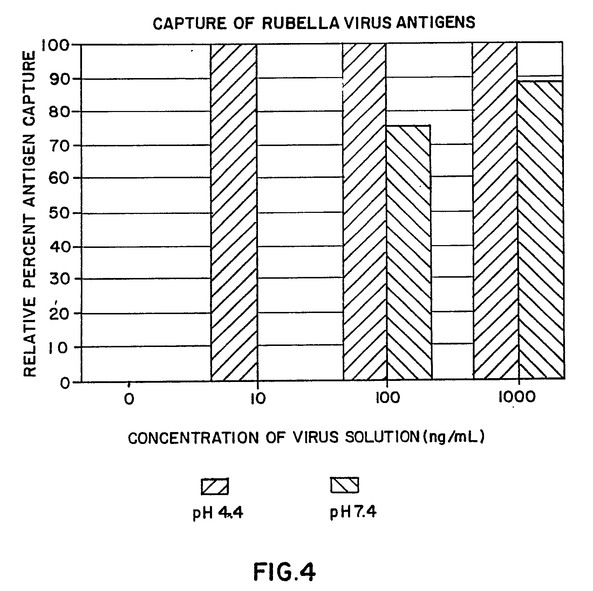

Figure 4 shows the improved ability of rubella specific antibodies absorbed on surfaces at pH 4.4 to capture rubella virus as compared with the same antibodies absorbed on the surface at pH 7.0. In this assay, surfaces coated with pH 4 antibodies were able to detect the presence of rubella virus in solution at 10 ng/ml, but surfaces coated with pH 7 antibodies were not.

The above Figures will be discussed in more detail in the following examples, which examples of the improved method for antigen capture essays according to the present invention, are given by way of illustration only and should not be construed in limitation thereof.

EXAMPLE I

Relative Surface Coverage by pH 4 and pH 7 Antibodies:

Antibodies were isolated from human plasma as "Cohn Effluent III" using the method described by J. L. Onclay, et al in the Journal of the American Chemical Society, vol. 71, pp 541-550, 1949. The antibodies obtained were of the IgG class and were >99.9% pure. Removal of the alcohol, concentration of the antibodies and formulation of the antibodies from the Cohn Effluent III was done under two different pH conditions. In one case the pH of an aliquot of Cohn Effluent III was adjusted to about 4.0 with HCl ;in the other case, an aliquot of Cohn Effluent III was adjusted to about pH 7.4 with NaOH. Final formulation of the antibodies obtained was done at 0.1% protein in water with 50 mM phosphate buffers. The final pH of the acid isolated antibodies was about 4.4 and was achieved using $H_2NaPO_4$. The final pH of the alkaline isolated antibodies was about 7.4 and was achieved using a mixture of $H_2NaPO_4$ and $HNa_2PO_4$. These antibody preparations were found to be >99.9IgG as measured by crossed immunoelectrophoresis and to have identical immunoglobulin class and subclass distributions as measured by radial immunodiffusion assay. Retention of antibodies from the starting Cohn Effluent III in the two preparations was >99% as measured by retention of $A_{280}$, thus insuring similar antibody composition between the two preparations.

Measurement of the ability of these two antibody preparations to absorb on plastic surfaces was done by applying various dilutions of the antibody solutions to the wells of commercial microtiter plates made of polystyrene. After letting the antibody solutions sit in contact with the surfaces for 48 hours, the solutions were removed and the wells washed six times with water.

Visualization and quantization of the amount of surface covered by the antibodies absorbed was done by allowing enzyme linked anti-human IgG antibody (raised in rabbit) in a 0.1albumin solution to react with surface absorbed human IgG. Since the enzyme linked anti-human IgG antibody binds preferentially to the human antibodies absorbed on the surface, the amount of enzyme retained by the surfaces is proportional to the amount of surface covered by the human antibodies.

Figure 2 shows the relative amount of surface covered by the two antibody solutions at different concentrations. These data show that a greater degree of surface coverage is obtained over a larger range of antibody solution concentrations from antibody solutions near pH4 than from antibody solutions in the physiological pH range. The data also show that antibody solutions formulated in the physiological pH range have a narrow absorption optimum which limits the working concentration of the antibody solution and the maximum area covered, while antibody solutions formulated near pH 4 do not. Thus, the absorption of antibodies isolated and formulated near pH4 allows greater flexibility and degree of surface coverage.

EXAMPLE II

Relative Capture of General Antigens at Two pHs:

Even though greater surface coverage can be achieved by using antibody preparations that have been isolated and/or formulated near pH4, it was not known if such antibodies after absorption would be better or worse at binding antigens than antibodies absorbed under conventional conditions. Therefore, experiments were done to assess this question by allowing Protein-A purified from staphylococcus aureus to bind to surfaces coated with the two antibody preparations. Since Protein-A will bind to all human IgG molecules, the amount of Protein-A captured by the immobilized antibodies is a measure of the relative binding ability of such antibodies. For these experiments the binding comparisons were made for surfaces covered by approximately the same degree by the two antibody preparations so that the comparison would reflect the relative binding affinity per molecule of antibody.

Visualization and quantization of the amount of Protein-A captured by the antibodies on the surfaces was done by allowing a solution of enzyme linked human IgG to react with the surface captured Protein-A. Results from these experiments are given in Figure 3, which shows that antibodies immobilized on the surface at pH near 4, were better able to bind Protein-A from solution than those antibodies immobilized from solution near pH 7. Of particular interest is the fact that antibodies immobilized on the surface near pH 4 showed increasingly better ability to capture Protein-A at the lower solution concentrations of this broad spectrum antigen.

EXAMPLE III

Relative Capture of Specific Antigens at Two pHs:

Since Protein-A binds to a unique domain of IgG, the Fc fragment, another set of experiments were done in order to evaluate the relative binding ability of the immobilized antibodies to the traditional antigen binding region of the antibodies, the Fab fragment. In these experiments inactivated rubella virus was used as the source of antigens, and the relative ability of the two types of immobilized antibodies to capture the virus from solution measured. Wells of polystyrene microtiter plates were coated to approximately the same degree with antibodies (in solution) isolated and formulated near pH4 and near pH7. The antibody solutions were then allowed to remain in contact with the surface of the wells until the microtiter plates were to be used. The solution contact time ranged from one day to one week. The antibody solutions were then removed and the excess antibody washed away with water. Then solutions of varying concentrations of rubella virus buffered in 100mM phosphate at pH7 were added to the wells. After shaking the plate for one to three hours at room temperature, the virus solutions were removed and the wells washed six times with water.

Meanwhile a solution of enzyme linked (labeled) antibodies to rubella virus, with 0.1% bovine albumin was prepared. The antibodies used for linkage to the enzyme had previously been isolated and formulated in the pH range 3.5 to 5.5 (accordingly to the present invention) and after coupling to the enzyme (by conventional methods at pH 7 for 4-5 hours) were stored at pH 4.5. Such solution of labeled antibodies was then buffered at pH 7, then added to the wells and allowed to mix for 1 to 3 hours, after which it was removed and the wells washed and the amount of retained enzyme activity measured,

The amount of enzyme activity retained was proportional to the amount of virus captured. Figure 4 shows the results of these experiments. Antibodies immobilized on the microtiter plate surfaces near pH4 were better able to capture rubella virus and virus antigens than antibodies immobilized on the surfaces in the physiologic pH range. At virus concentrations of 10ng/ml only the surfaces coated with antibodies near pH4 could detect the presence of the virus. It took a hundred-fold increase in virus concentration before the surface coated with antibodies in the physiologic pH range showed results comparable to those of antibodies coated near pH4.

The above examples indicate that antibodies absorbed on surfaces from solutions in the pH range 3.5 to 5.5 are stable with regard to their improved ability to bind antigens and because of this improved ability, this invention prefers to use enzyme linked (labeled) antibodies that have been isolated and formulated in the acidic pH range at the pH range 3.5 to 5.5. This is because, it has been found that as in the case of the original antibody formulation above, considerably more thorough labeled antibody binding to the so-bound antigens results in both the low and high pH assays and gives a more sensitive and accurate reading of antigen capture in each of the samples of the present example. That is, it has been found that labeled antibodies formulated from solutions at pH 3.5 to 5.5 bind with antigens in markedly greater numbers (on the order of about 2 to 1) than labeled antibodies formulated from conventional solutions at about pH 7. This marked increase in sensitivity is added to the even greater increase (in sensitivity) in antibody coating of plastic surfaces and the resulting antigen capture, as embodied in the present invention and shown in the graphs of Figures 2, 3 and 4.

Note that though labeled antibodies are advantageously formulated (in solution) in the pH range 3.5 to 5.5 according to the present invention, they may be desirably coupled to markers, e.g. enzymes at higher pH for a few hours and then the pH of the enzyme-linked antibodies (solution) is lowered to a pH range of

6

3.5 to 5.5 for storage, with little or no discernable reduction in their improved antigen binding ability, i.e. improved detection sensitivity by the labeled antibodies will result as long as the exposure of such antibodies to a coupling solution at elevated pH (eg. at pH 5.5 to 9.5 or higher) is but for a relatively short duration.

In addition, the present invention demonstrates that antibody absorption on plastic surfaces is significantly improved when antibodies (isolated and) formulated in a range of 3.5 to 5.5 are used.

When this is done as discussed above, more antibodies are absorbed on plastic surfaces, which antibodies have an improved ability to capture antigens, a double improvement, thus significantly improving the sensitivity of the test for a particular antigen, including that of a virus.

The invention further includes the improved antibody-coated surfaces that result from such method, as well as the improved labeled antibodies,

Also, as discussed above, in Example II, the stability of the antibody solutions at pH 3.5 to 5.5 is enhanced by maintaining such solutions in the wells until just before time for use in combination with antigen solutions.

According to the present invention, antibodies can be immobilized on various surfaces, including glass or on various plastic surfaces e.g. by hydrophobic bonding, including on thermoplastics and thermoset plastics, which include polyolefins, including polyethylene, polystyrene, ABS and various other plastics.

## Claims

1. A method for improving antigen capture assays comprising formulating antibodies in the pH range of 3.5 to 5.5 and then coating said antibodies on at least one surface in said pH range.

2. A method of claim 1, wherein the antibody coated surface is then contacted with an antigen solution, so that antigens bind to a plurality of said antibodies on said surface to form an antibody-antigen coating thereon.

3. A method of claim 2, wherein a solution of labeled antibodies is then contacted with the so-coated surface to bind with the so-bound antigens, so that a plurality of the captured antigens can be measured, optionally said labeled antibodies including antibodies linked to markers selected from enzymes, radioactive isotopes and fluorescent molecules.

4. A method of any one of claims 1 to 3 wherein said antibodies are isolated and formulated in the pH range of 3.5 to 5.5, or wherein said antibodies have been isolated above pH 5.5 and then promptly formulated in the pH range 3.5 to 5.5.

5. A method of any one of claims 1 to 4 wherein said antibodies are absorbed from solution onto plastic surfaces at a pH between 3.5 and 5.5 so as to coat said surfaces with said antibodies.

6. A method of any one of claims 1 to 5 wherein said antibody coated surface is contacted with antigens in solution at pH above 5.0 to capture same.

7. A method for improving antigen capture assays comprising contacting antibodies formulated in the pH range of 3.5 to 5.5 to markers to form labeled antibodies of improved sensitivity.

8. A method of claim 7 wherein said labeled antibodies are contacted in solution with antigens bonded to antibodies coated on an underlying surface.

9. A method for improving antigen capture assays comprising formulating antibodies in solution in the pH range of 3.5 to 5.5, applying the resultant solution to a plastic surface to coat said surface with antibodies and washing the so-coated surface to provide improved surface coverage of absorbed antibodies, optionally said antibody solution being maintained in contact with the surface to be coated until just before the so-coated surface is to be washed and contacted with antigens.

10. A method of claim 9 wherein the so-coated surface is then contacted with an antigen solution, a plurality of the antigens bind to said antibodies and labeled antibodies are contacted in turn with the so-bound antigens to visualize or quantitate a plurality of said antigens.

11. A method for preparing labeled antibodies wherein antibodies formulated in the pH range of 3.5 to 5.5 are coupled with markers in the pH range of 6.5 to 9.5 to form labeled antibodies, after which the pH is reduced to the pH range of 3.5 to 5.5 for storage until needed.

12. A method of claim 11 wherein the so-formed labeled antibodies are contacted with antigens which are bonded to other antibodies immobilized on a plastic surface.

13. Antibody-coated surfaces comprising surfaces, preferably plastic, absorbed with antibodies, which antibodies were formulated and absorbed in the pH range 3.5 to 5.5.

14. Antibody coated surfaces of claim 13 bonded in turn to antigens.

7

15. Antibody coated surfaces of claim 14 wherein said antigens are bonded in turn to labeled antibodies, optionally said labeled antibodies comprising antibodies formulated in the pH range 3.5 to 5.5 and thereafter linked to markers.

16. Labeled antibodies comprising antibodies formulated in the pH range 3.5 to 5.5 and thereafter linked to markers, optionally markers selected from enzymes, radioactive isotopes and fluorescent molecules.

FIG.1a

FIG.1b

FIG.1c

SURFACE COVERAGE BY pH4 & pH7 ANTIBODY

FIG. 2

CAPTURE OF PROTEIN-A FROM SOLUTION

RELATIVE PERCENT BINDING OF PROTEIN-A

PROTEIN-A SOLUTION CONCENTRATION ( μg /mL)

pH 4.4          pH 7.4

FIG.3

CAPTURE OF RUBELLA VIRUS ANTIGENS

FIG.4